# EUROPÄISCHE PATENTSCHRIFT

(11) **EP 0 684 840 B1**
(45) Veröffentlichungstag und Bekanntmachung des Hinweises auf die Patenterteilung: **23.08.2000**
(21) Anmeldenummer: 94906226.9
(22) Anmeldetag: 07.02.1994
(51) Int. Cl.: A61K 47/24, A61K 47/14, C07C 69/40, C08G 65/32

(54) **VERFAHREN ZUR HERSTELLUNG KOLLOIDALER WÄSSRIGER LÖSUNGEN SCHWERLÖSLICHER WIRKSTOFFE UND EIN LIPID DAFÜR**
METHOD OF PREPARING COLLOIDAL AQUEOUS SOLUTIONS OF ACTIVE SUBSTANCES WHICH ARE ONLY SLIGHTLY SOLUBLE IN WATER, AND A LIPID FOR USE IN THE METHOD
PROCEDE DE FABRICATION DE SOLUTIONS AQUEUSES COLLOIDALES DE MATIERES ACTIVES DIFFICILEMENT SOLUBLES, ET LIPIDE UTILISE POUR CE PROCEDE

(30) Priorität: 18.02.1993 DE 4305004
(43) Veröffentlichungstag der Anmeldung: 06.12.1995
(73) Patentinhaber: KNOLL AKTIENGESELLSCHAFT, 67061 Ludwigshafen (DE)
(72) Erfinder: ROSENBERG, Joerg, D-67158 Ellerstadt (DE); ROMERDAHL, Cynthia, Wayland, MA 01778 (US); GRUENHAGEN, Hans-Heinrich, D-67071 Ludwigshafen (DE)
(74) Vertreter: Bieberbach, Andreas, Dr.
(86) Internationale Anmeldenummer: EP9400333
(87) Internationale Veröffentlichungsnummer: WO9419019

(56) Entgegenhaltungen:
- EP-A- 0 317 120
- EP-A- 0 366 990
- EP-A- 0 456 106
- DE-A- 3 010 041
- DE-A- 4 002 165
- US-A- 3 960 935

## Beschreibung

Die Verabreichung eines Arzneistoffs durch z.B. Injektion oder Infusion in die Blutbahn scheitert oft an dessen Schwerlöslichkeit in wäßrigen Systemen. Es sind daher in den letzten Jahren verschiedene Verfahren entwickelt worden, um mit Hilfe geeigneter Löslichkeitsvermittler den Arzneistoff in einer wäßrigen Phase zu formulieren.

Die meisten Verfahren nutzen dabei den lösungsfördernden Effekt von Detergentien oder Emulgatoren aus (vgl. Voigt: Lehrbuch der pharmazeutischen Technologie 5. Aufl. Verlag Chemie. S. 334). Obwohl die Herstellung solcher Lösungen sehr einfach ist und eine Vielzahl von Arzneistoffen damit solubilisiert werden können, bereitet die von den Detergentien ausgehende Toxizität oft erhebliche Probleme.

Eine weitere Möglichkeit besteht darin, die Arzneistoffe mit Hilfe von ringförmigen Kohlenhydraten (Cyclodextrinen) zu versetzen, die den Arzneistoff komplexieren (Pharm. Techn. Intern., Februar 1991, S. 15) und die dadurch die Löslichkeit oft entscheidend verbessern. Da der innere Hohlraum der Cyclodextrine aber begrenzt ist, lassen sich viele Moleküle aus sterischen Gründen nicht komplexieren, obwohl die Cyclodextrine toxikologisch günstiger zu bewerten sind als die Detergentien.

Als weitere Klasse von Löslichkeitsvermittlern kommen die Phospholipide infrage, die als körpereigene Moleküle (sie sind Bestandteil einer jeden Zellmembran) eine sehr gute Verträglichkeit auch in Injektionslösungen zeigen. Der Einsatz von Phospholipiden scheitert aber oft daran, daß diese in wäßrigen Systemen nicht molekular, sondern nur unter Bildung kolloidaler Aggregate löslich sind. Diese Kolloidteilchen sind beim einfachen Dispergieren von Phospholipiden in wäßrigen Lösungen jedoch so groß, daß solche phospholipidhaltigen Lösungen für Injektions- und Infusionszubereitungen im allgemeinen nicht einsetzbar sind (Emboliegefahr). Die durch Suspendieren von Phospholipiden in wäßrigen Systemen erhältlichen grobkolloidalen trüben Lösungen müssen daher vor ihrer Verwendung erst mit Hilfe geeigneter Verfahren homogenisiert werden, so daß die Größe der Partikel in den Lösungen auf Werte erniedrigt wird, die einen Einsatz der Phospholipide in Injektions- oder Infusionslösungen erlauben (vgl. US-A 5 008 050). Nur in ganz wenigen Fällen gelingt es bei ausgewählten Arzneistoffen, direkt aus Wirkstoff und Phospholipid in wäßrigen Systemen zu injizierbaren Arzneistoffzubereitungen zu gelangen, ohne daß durch relativ aufwendige Homogenisierverfahren erst eine Verkleinerung der kolloidalen Partikel erfolgen muß.

Der größte Teil aller bekannten Arzneimittelwirkstoffe ist schwach basisch, d.h. er besitzt eine protonierbare Aminogruppe. Solche schwach basischen Substanzen sind oft durch Bildung von Salzen mit geeigneten organischen oder anorganischen Säuren in Wasser ausreichend löslich. Die Salze zeigen allerdings im allgemeinen nur im sauren Milieu eine ausreichende Löslichkeit. Solche pH-Werte sind für pharmazeutische Injektionszubereitungen nicht akzeptabel. Beim Versuch, neutrale pH-Werte einzustellen, fallen die Wirkstoffe in erheblichem Maße aus, so daß meist nur relativ geringe Konzentrationen bei physiologischen pH-Werten erreichbar sind oder auf Cosolventien zurückgegriffen werden muß, aus denen die Wirkstoffe nach der Injektion ebenfalls aber oft wieder ausfallen (J. Pharm. Sci. 72, S. 1014 (1983).

Es wurden nun Stoffe gefunden, die sich gut zum In-Lösung-Bringen von schwer löslichen Wirkstoffen eignen.

Gegenstand der Erfindung sind wasserlösliche Lipide der Formel I 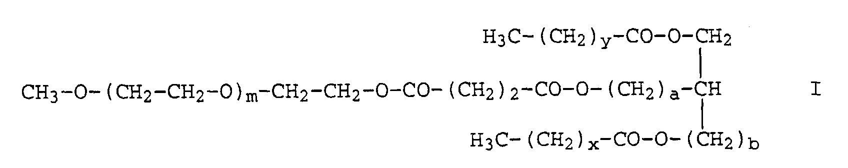 worin m eine Zahl zwischen 40 und 500, vorzugsweise zwischen 40 und 200, eine der Zahlen a und b 0 und die andere 1, x eine Zahl von 10 - 18, vorzugsweise 12, 13 oder 14, und y eine Zahl von 10 - 18, vorzugsweise 12, 13 oder 14, sind.

Besonders bevorzugt sind Verbindungen der Formel I, in denen x und y identisch sind und 12, 13 oder 14 bedeuten.

Die neuen Lipide lassen sich herstellen aus einer Verbindung der Formel 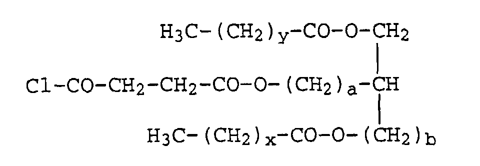 durch Kondensation mit einem Polyethylenglykolmonomethylether (M = 1.800-22.000, vorzugsweise etwa 5000) der Formel

CH₃-O-(CH₂-CH₂-O)ₘ-CH₂-CH₂-OH

in Gegenwart einer Base wie Pyridin.

Zum In-Lösung-Bringen wird der Wirkstoff mit einem Phospholipid und einem wasserlöslichen Lipid in einem organischen Lösungsmittel gelöst, die so erhaltene Lösung zur Trocknen eingeengt, der Rückstand in einem stark sauren Puffer aufgenommen und bei erhöhter Temperatur gerührt, bis eine kolloidale Lösung entstanden ist, die kolloidale Lösung abgekühlt und auf einen pH-Wert von 6 - 7 eingestellt und sterilisiert.

Für das neue Verfahren kommen insbesondere Wirkstoffe in Betracht, die normalerweise in Wasser schwer löslich sind, die sich aber in Gegenwart von Säuren lösen. Beispiele für solche Substanzen sind insbesondere Wirkstoffe mit protonierbaren Aminogruppen im Molekül, d.h. alle basischen Arzneistoffe, deren Löslichkeit in demineralisiertem Wasser auch in Form der Salze im Mittel unter 1 mg/ml liegt.

Sollen Wirkstoffe in Lösung gebracht werden, die schwer löslich sind und sich auch nicht in Säuren lösen, so muß man den Anteil des wasserlöslichen Lipids im Verhältnis zum Phospholipid erhöhen.

Als Phospholipidkomponente können alle natürlichen bzw. synthetisch hergestellten Phospholipide verwendet werden; auch Mischungen verschiedener Phospholipide sind möglich. Besonders bevorzugt sind die aus Eigelb oder Soja isolierten Lecithine, insbesondere dann, wenn sie mehr als ca. 80 % Phosphatidylcholin enthalten.

Als wasserlösliche Lipide sind übliche pharmazeutisch gebräuchliche Lipide einsetzbar. Hierzu zählen insbesondere folgende in den Arzneibüchern aufgeführten Substanzen: Fettsäure-Salze (Natriumstearat/-palmitat); Partialglyceride (Glycerinmonostearat/-oleat, acetylierte Monoglyceride); Zuckerester (Sorbitanmonolaurat/-oleat/-palmitat/-stearat/-tristearat); Salze von Sulfonsäuren (Natrium-cetylstearylsulfat/-laurylsulfat, -tetradecylsulfat); Ethoxylate von Fettsäuren/Glyceriden/Zuckerestern (Macrogolstearat 400, Polyoxyl-40/50-stearat, Polyoxyl-10-Oleyl Ether, Polyoxyl-20-Cetostearyl Ether, Polydocanol 600, Macrogol-Glycerolhydroxystearat, Macrogol-1000-Glycerolmonolaurat/-oleat/-stearat/-triricinoleat, Polysorbat 20/40/60/80; sowie Poloxamere.

Daneben erwies sich insbesondere für das In-Lösung-Bringen von den Bisnaphthalimiden der US-PS 4,874,683 ein neues synthetisches wasserlösliches Lipid der Formel I als sehr gut geeignet.

Das Gewichtsverhältnis Phospholipid : wasserlösliches Lipid in den Formulierungen kann von 10:1 bis 1:1 betragen. Das für den jeweiligen Wirkstoff bestgeeignete Verhältnis muß empirisch ermittelt werden.

Zur Einstellung und Konstanthaltung der pH-Werte muß die Lösung eine geeignete Puffersubstanz enthalten. Besonders bevorzugt ist hierfür Phosphorsäure, da diese die notwendigen sehr niedrigen anfänglichen pH-Werte stabil einzustellen erlaubt.

Es zeigte sich, daß für das Gelingen der Präparationen nach dem erfindungsgemäßen Verfahren anfangs pH-Werte von unter 2 vorzuziehen sind. Somit kommen Puffersubstanzen in Frage, mit denen sich diese sauren pH-Werte einstellen lassen. Der verwendete Puffer sollte gleichzeitig eine ausreichende Pufferkapazität auch im physiologischen pH-Bereich zwischen pH 6 und 7 besitzen, um nach der Titration der Lösung in diesem pH-Bereich den pH ohne Schwankungen konstant zu halten. Als gut geeignet haben sich Phosphorsäurelösungen in einer Konzentration von etwa 0,05 bis 0,15 mol/l erwiesen. Besonders günstig ist eine Konzentration an Phosphorsäure von 0,14 mol/l. Solche Lösungen besitzen in der Regel einen pH-Wert im optimalen Bereich von etwa pH 1,5 und ergeben beim anschließenden Titrieren mit verdünnter Natronlauge pH-stabile Lösungen im physiologischen pH-Bereich von ca. pH 7, die zudem meist blutisoton sind (ca. 280 mosmol/kg), so daß sich eine weitere Isotonisierung der Lösungen erübrigt.

Die schwer löslichen Substanzen werden mit einem Phospholipid und dem wasserlöslichen Lipid in stark saurer wäßriger Lösung gemischt. Durch einfaches Rühren gelingt es bereits, die Substanzen zu lösen oder zu emulgieren, ohne daß ein besonderes Homogenisierverfahren angewendet werden muß. Nach Einstellen des pH-Wertes auf 6 - 7 erhält man eine stabile Wirkstofflösung bzw. -emulsion.

Besonders gut gelingt es, das Substanzgemisch in Lösung zu bringen, wenn man das Gemisch zuvor durch Lösen in einem geeigneten organischen Lösungsmittel und anschließendes vollständiges Entfernen des Lösungsmittels miteinander vermischt.

In vielen Fällen zeigte es sich, daß es günstig ist, die Solubilisierung anfangs in einer nur geringen Menge der (stark sauren) Pufferlösung ablaufen zu lassen (ca. 10 % der insgesamt benötigten Pufferlösung). Nach erfolgter Solubilisierung und pH-Titration wird dann auf das vorgesehene Endvolumen aufgefüllt.

Das Einengen zur Trockne kann durch Destillation oder Gefriertrocknung erfolgen. Besonders günstig ist es, bei kleinen Ansätzen die Lösung durch Rotationsverdampfen einzuengen, da man hierbei die Substanz in Form eines Films an der Innenseite des Verdampfers erhält, wodurch die anschließende Hydratation erleichtert bzw. beschleunigt wird.

Zum Trockenrückstand gibt man dann eine abgemessene Menge der stark sauren Pufferlösung und rührt bei erhöhter Temperatur so lange, bis sich eine homogene kolloidale Lösung gebildet hat, die frei von grobkolloidalen Aggregaten ist. Diese Lösung kühlt man auf Raumtemperatur ab und gibt unter Rühren eine wäßrige Lösung einer Base zu, bis ein pH-Wert von etwa 6 - 7 erreicht ist. Die fertige Lösung kann dann noch stufenweise über Membranfilter bis zur Porengröße 0,2 µm (Sterilfiltration) filtriert, d.h. entkeimt werden.

Wie die in den Beispielen enthaltenen Daten zeigen, wird durch die Kombination der beschriebenen Schritte die Löslichkeit von schwer löslichen Arzneimittelwirkstoffen so stark verbessert, daß diese in wäßrigen Lösungen appliziert werden können, was für deren therapeutischen Anwendung ein großer Vorteil ist.

Ein weiterer Vorteil der neuen Lösungen besteht darin, daß in ihnen die Toxizität der Wirkstoffe verringert ist, so daß deren Dosierung um bis zu einem Faktor 2 erhöht werden kann, ohne daß unerwünschte Nebenwirkungen auftreten. Das ist besonders wichtig für die Anwendung von Cancerostatica.

### Beispiel 1

In der US-PS 4,874,683 sind Wirkstoffe beschrieben, die eine Bisnaphthalimid-Struktur besitzen. Wegen der sehr schlechten Wasserlöslichkeit ist die Herstellung ausreichend konzentrierter wäßriger Wirkstofflösungen sehr erschwert. Unter Zuhilfenahme von Löslichkeitsvermittlern wie Dimethylsulfoxid sind zwar Injektionslösungen herstellbar, aus diesen flockt aber nach der Injektion im Blut der Wirkstoff schnell wieder aus.

Mit einem Derivat dieser Bisnaphthalimide 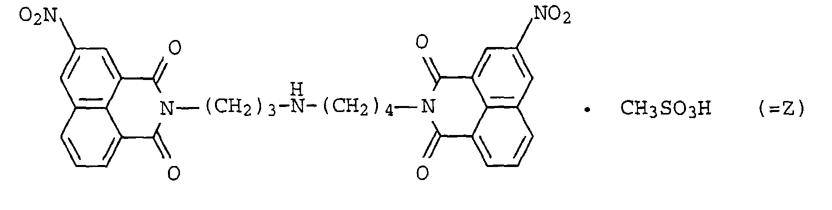 wurde die Löslichkeit wie folgt in wäßrigen Formulierungen untersucht:

### Versuch A (Vergleichsversuch)

40 mg Z wurden in 10 ml Methylenchlorid/Methanol (9 + 1) gelöst. Aus der Lösung wurde am Rotationsverdampf er das Lösungsmittel wieder restlos im Vakuum entfernt. Zum Rückstand gab man 8 ml einer 0,14 molaren Phosphorsäurelösung (pH ≈ 1,5) und drehte den Kolben 1 h am Rotationsverdampfer (ohne Vakuum) bei 50°C Wasserbadtemperatur. Ein erheblicher Teil des Wirkstoffs konnte so in Lösung gebracht werden. Nach dem Abkühlen auf Raumtemperatur titrierte man mit 2 molarer Natronlauge auf einen pH von 6,8 und füllte auf 10 ml mit Phosphatpuff er pH 6,8 auf. Dabei fiel der Wirkstoff zum größen Teil aus. Der klare Überstand wurde durch ein 0,2 µm Spritzen-Sterilfilter filtriert und in dieser Lösung photometrisch die Wirkstoffkonzentration bestimmt. Der Wirkstoffgehalt dieser Lösung betrug 0,04 mg/ml.

### Versuch B (Vergleichsversuch)

Der Versuch erfolgte analog Versuch A, aber es wurden zusätzlich zum Wirkstoff 50 mg des neuen wasserlöslichen Lipids I-a eingewogen. Nach der Entfernung des Lösungsmittels gab man 10 ml einer mit 2 molarer Natronlauge auf pH 6,8 titrierten 0,14 molaren Phosphorsäurelösung zu und rührte ebenfalls 1 h im Wasserbad am Rotationsverdampfer bei 50°C.

Der Wirkstoffgehalt der titrierten Lösung (0,2 µm-Filter) betrug 0,04 mg/ml.

### Versuch C (Vergleichsversuch)

Der Versuch erfolgte analog Versuch B, aber statt des neuen wasserlöslichen Lipids I-a kamen 250 mg Phospholipid zum Einsatz (Eierlecithin E 100, Fa. Lipoid KG, Ludwigshafen).

Der Wirkstoffgehalt der filtrierten Lösung betrug 0,04 mg/ml.

### Versuch D (Vergleichsversuch)

Der Versuch erfolgte analog Versuch A, aber zusätzlich zum Wirkstoff wurden 50 mg des wasserlöslichen Lipids I-a eingewogen.

Der Wirkstoffgehalt der filtrierten Lösung betrug 0,08 mg/ml.

### Versuch E (Vergleichsversuch)

Der Versuch erfolgte analog Versuch B, aber zusätzlich zum Wirkstoff und dem wasserlöslichen neuen Lipid I-a wurden 250 mg Phospholipid (Eierlecithin E 100) eingewogen.

Der Wirkstoffgehalt der filtrierten Lösung betrug 0,1 mg/ml.

### Versuch F (Vergleichsversuch)

Der Versuch erfolgte analog Versuch A, aber zusätzlich zum Wirkstoff wurden 250 mg Phospholipid (Eierlecithin E 100) eingewogen.

Der Wirkstoffgehalt der filtrierten Lösung betrug 2,8 mg/ml.

### Versuch G

Der Versuch erfolgte analog Versuch F, aber zusätzlich wurden 50 mg des neuen wasserlöslichen Lipids I-a eingewogen. Die Filteroberfläche des Sterilfilters war bei diesem Versuch im Gegensatz zu den Beispielen 1 - 6 frei von ungelösten Wirkstoff-Resten.

Der Wirkstoffgehalt der filtrierten Lösung betrug 4,0 mg/ml.

Das für die Versuche B, D, E und G verwendete neue wasserlösliche Lipid I-a wurde wie folgt hergestellt:

35,00 g Polyethylenglycolmonomethylether (MW ca. 5000) wurden in 70 ml Dichlormethan zusammen mit 0,71 ml Pyridin gelöst. Dazu gab man unter Rühren 4,81 g 1,2-Dipalmitoylglycero-3-(3-chlorformyl)-propionat, gelöst in 5 ml Dichlormethan, unter Rühren zu. Der Ansatz wurde 48 h am Rückfluß erhitzt und anschließend erneut 4,81 g 1,2-Dipalmitoylglycero-3-(3-chlorformyl)-propionat zugegeben. Es wurde weitere 24 h am Rückfluß erhitzt. Das Lösungsmittel wurde dann im Vakuum entfernt, der Rückstand mit 500 ml Diethylether verrührt. Diese Suspension saugte man über eine breite Nutsche ab. Den Nutschenrückstand löste man in 300 ml Dichlormethan und schüttelte vorsichtig (Emulsionsbildung) einmal mit 100 ml Salzsäure (1 molar) und zweimal mit je 100 ml Wasser aus. Die organische Phase wurde mit Na₂SO₄ getrocknet und eingedampft. Zum Rückstand gab man 785 ml Ethanol, löste in der Wärme und ließ im Eisbad kristallisieren. Der Niederschlag wurde abgesaugt, mit eiskaltem Ethanol gewaschen und getrocknet.
- Ausbeute:: 37,84 g farbloses Pulver (95 %)
- Schmelzbereich:: 55-59°C

Analog wurden folgende Verbindungen der Formel I hergestellt.
- Ib: a=0, b=1, x=y=14, m etwa 40-45,
farbloses Pulver, Schmelzbereich 50-52°C,
Ausbeute 94 %;
- Ic: a=0, b=1, x=y=12, m etwa 100-120,
farbloses Pulver, Schmelzbereich 57-58°C,
Ausbeute 95 %;
- Id: a=1, b=0, x=y=14, m etwa 100-120,
farbloses Pulver, welches aus Essigsäureethylester/Diisopropylether (4:6) kristallisiert wurde,
Schmelzbereich 70-70°C, Ausbeute 81 %.

### Beispiel 2

35 mg β-Carotin wurden in einem 100-ml-Rundkolben zusammen mit 500 mg Eierlecithin E 100 (Fa. Lipoid KG, Ludwigshafen) und 500 mg Cremophor RH-40® (BASF) in einer Mischung aus 30 ml Cyclohexan und 5 ml Methanol gelöst. Das Lösungsmittel wurde dann im Vakuum-Rotationsverdampfer restlos entfernt, so daß ein dünner roter Film auf der Glaskolbenwand zurückblieb. Zu diesem Rückstand gab man 18,5 ml einer 0,14 molaren Phosphorsäurelösung (pH - 1,5). Den Kolben drehte man dann bei einer Wasserbadtemperatur von 50°C bis der gesamte Film von der Glaswand abgelöst war (15 min). Diese Lösung titrierte man bei Raumtemperatur mit 2 molarer Natronlauge unter Rühren auf pH 7. Der Wirkstoffgehalt lag bei 1,70 mg/ml. Die Lösung konnte stufenweise über 1,2 µm, 0,45 µm und 0,2 µm Filter sterilfiltriert werden.

Führte man das Beispiel 2 analog Beispiel 1, Versuchen A, B, C, D, E und F durch, so lag der Wirkstoffgehalt der Lösung unter 0,25 mg/ml.

## Patentansprüche

1. Wasserlösliches Lipid der Formel 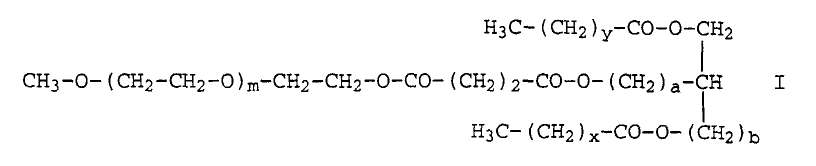 worin
m eine Zahl zwischen 40 und 500,
eine der Zahlen a und b 0 und die andere 1,
x eine Zahl von 10 bis 18 und
y eine Zahl von 10 bis 18
sind.

2. Verwendung des Lipids gemäß Anspruch 1 zum In-Lösung-Bringen von schwer löslichen Wirkstoffen.

## Claims

1. A water-soluble lipid of the formula 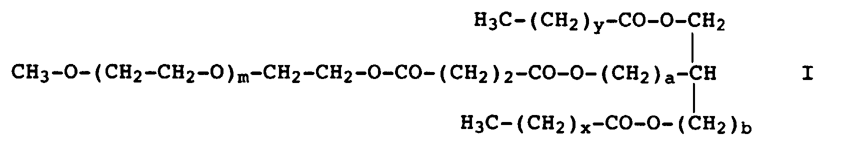 where
m is a number from 40 to 500,
one of the numbers a and b is 0 and the other is 1,
x is a number from 10 to 18 and
y is a number from 10 to 18.

2. The use of the lipid as claimed in claim 1 for producing solutions of active substances of low solubility.

## Revendications

1. Lipide hydrosoluble de formule 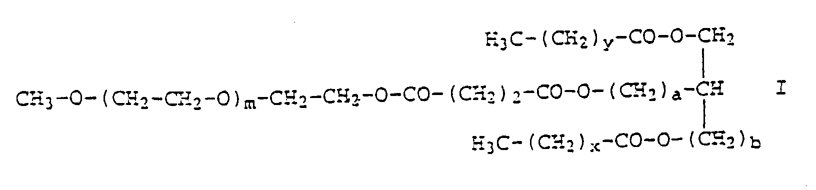 où
m est un nombre entre 40 et 500,
l'un des nombres a et b est 0 et l'autre est 1,
x est un nombre de 10 à 18, et
y est un nombre de 10 à 18.

2. Utilisation du lipide selon la revendication 1 pour la mise en solution de substances actives difficilement solubles.
